# EUROPEAN PATENT SPECIFICATION

(11) **EP 0 372 612 B1**
(45) Date of publication and mention of the grant of the patent: **05.01.1994**
(21) Application number: 89202972.9
(22) Date of filing: 23.11.1989
(51) Int. Cl.: C08G 77/26, C08G 77/16, C08G 77/388, A61K 7/16

(54) **Hydroxylhydrocarbyl-modified aminoalkyl silicones**
Hydroxylhydrocarbyl-modifizierte Aminoalkylsilicone
Aminoalcoylsilicones modifiés par des groupes hydroxylhydrocarbyliques

(30) Priority: 28.11.1988 US 276726
(43) Date of publication of application: 13.06.1990
(73) Proprietor: UNILEVER N.V., 3013 AL Rotterdam (NL); UNILEVER PLC, London EC4P 4BQ (GB)
(72) Inventor: Lin, Samuel, Paramus New Jersey 07652 (US); Parriott, Colleen, Monroe New York 10950 (US)
(74) Representative: van Gent, Jan Paulus

(56) References cited:
- WO-A-79/00454
- US-A- 3 389 160
- US-A- 4 680 366
- CHEMICAL ABSTRACTS vol. 90, no. 16, 16 April 1979, page 612, abstract no. 130686x, Columbus, Ohio, US; & JP - A - 78135621

## Description

### FIELD OF THE INVENTION

The application concerns novel aminoalkylsilicone compounds which are useful in dentifrices and other consumer products.

### BACKGROUND OF THE INVENTION

Great strides have been made in recent years in the field of oral health care. However, much remains to be done. While the development of anticaries agents, especially the fluorides, has led to a decline in the incidence of tooth caries it is desirable to even further decrease the number of teeth affected thereby. Moreover, attention in the oral health care field has increasingly focused on the problems of gum disease, periodontitis. While antibacterial agents have been proposed for inclusion in products for use by consumers in the treatment of periodontitis, certain problems have been associated with their use. For example, use of chlorhexidine, which has been known as as antibacterial agent, has been associated with staining problems; it produces yellow to dark brown stains on teeth, tongue and oral mucosa. Furthermore, chlorhexidine has a very bitter taste.

There has been a need, therefore, for developing a dentifrice formulation including chlorhexidine or other antimicrobial agents such as quaternary ammonium antibacterial compounds, which does not cause staining of the teeth and which has an improved taste. Moreover, dentifrices having improved anticavity effects are also desirable.

Staining can be troublesome, whether or not chlorhexidine is the cause. The accumulation of stains on tooth surfaces poses an esthetic problem for many individuals.

Plaque is a common factor in caries, gum disease and staining and greatly contributes to their development. Proper oral hygiene as currently practiced requires that plaque be removed or prevented not only for cosmetic purposes but also to climinate a source of potential injury to teeth and gums.

Silicones have previously been suggested for inclusion in dentifrice compositions in that it has been proposed that they would coat the teeth and thereby prevent cavities and staining. For example, British Patent Specification 689,679 discloses a mouthwash containing an organopolysiloxane for the purpose of preventing adhesion of, or for removal of, tars, stains, tartar and food particles from the teeth. However, polymers such as those disclosed in the '679 specification, have not generally been successfully used for coating the teeth since it has been found that the polysiloxane does not adhere to the teeth for prolonged periods of time. Therefore, the need for dentifrice formulations including a hydrophobic substance which effectively coats the teeth has not been satisfied.

Viccaro, et al. copending application EP-A-0 373 688 filed simultaneous herewith and entitled "Dentifrices Containing Aminoalkyl Silicones" discloses dentifrice formulations including aminoalkylsilicones for coating the teeth and inhibiting stain and caries. The aminoalkylsilicones have been found to be more substantive than alkylsilicones, apparently due to the interaction of the positively charged nitrogen of the amine with the negative charges on the surface of the teeth. However, amine groups tend to react with certain chemical groups found in toothpaste components such as the aldehydes of flavoring ingredients. Consequently, aminoalkylsilicones bearing amine groups capable of being protonated over a broad pH range yet of reduced reactivity are desirable.

Silicones have been used or proposed for use in many consumer products other than dentifrices. Where silicones are included to coat charged objects, such as furs, aminoalkylsilicones will be of use. Like dentifrices, these products may contain compounds having moieties which tend to react undesirably with amine groups. Therefore, aminoalkylsilicones in which the amine group is somewhat deactivated would be useful in these applications, as well.

Attempts have been made to modify the structure of aminosilicones to decrease the reactivity of the amino groups. In U.S. Patent No. 4,507,455 aminosilicones are reacted with acetic anhydride to form amides. It is believed that the modifications seriously limit the pH range over which the amines will be protonated thereby detracting from the usefulness of the respective aminoalkylsilicones in applications, such as those mentioned above, wherein protonation of the amine is important. Also, U.S. Patent No. 4,472,566 discloses the reaction of aminosilicones with benzylchloride to yield secondary and tertiary amines.

Morehouse U.S. Patent No. 3,032,577 discloses organosiloxanes which are said to be useful for a variety of applications in the synthetic polymer art, particularly as flocculating agents for aqueous dispersion of clay. The organosiloxanes of the Morehouse patent include units of the formula: 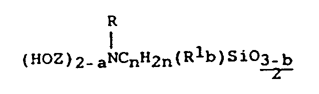
wherein -OZ- is the divalent group derived from a monoepoxide by opening of the oxirane ring, HO is interconnected to N through 2 carbon atoms, a is an integer from 0 to 1, n is an integer from 3 to 15, and R may be hydrogen, monovalent hydrocarbon or 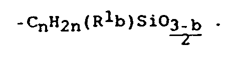
Morehouse does not appear to suggest that his compounds would be useful in dentifrices and the like, nor does he seem to recognize the desirability of using compounds which can yield a higher positive charge density.

### SUMMARY OF THE INVENTION:

We have now discovered a novel class of modified aminosilicones which have reduced reactivity but still retain positive charges over a broad pH range. The invention also embraces the process of making the components, and compositions employing them. The silicones of this invention are produced by treating silicones containing primary or secondary amine functional groups with epoxides such as ethylene oxide. This reaction effectively converts most amines to tertiary amines with one or two beta-hydroxylhydrocarbyl substituents. The tertiary molecular structure and the electron withdrawing property of beta-hydroxyls reduce the amine reactivity, but still maintain the pKa between seven and nine. Thus, in most formulation conditions, these novel silicones remain as cationic polymers and have good physical interactions with the substrate surface but show reduced chemical reactivities toward flavoring agents, dyes and skin.

The novel silicones of the invention comprise an organosiloxane including at least one unit of formula A: 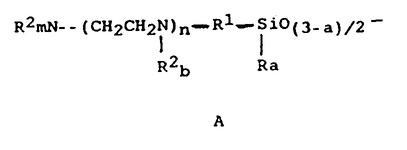
wherein
a is from 0 to 2, n is from 1 to 5, R is a monovalent radical,
R¹ is a divalent hydrocarbon radical, R² is 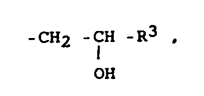
R³ is selected from the group consisting of hydrogen and monovalent hydrocarbon radicals, m is 1 or 2, and b is 0 or 1, whereby when m = 1 and/or b = the remainder of the valences on the N-atoms are satisfied by H-atoms.

R is selected from hydrocarbon radicals, halogenated hydrocarbon radicals, hydrogen, hydroxyl, and alkyoxyl groups. Especially preferred are methyl, phenyl and -trifluoropropyl. R groups having from 1 to 10 and particularly 1 to 4 carbon atoms are preferred. The divalent hydrocarbon radical of R¹ preferably includes from 1 to 20 carbon atoms, preferably 3 to 20 carbon atoms. Preferred R³ groups include hydrogen, hydrocarbon radicals, methyl or phenyl. Hydrogen and methyl are particularly preferred. Where R³ is a hydrocarbon radical, it is preferred that the radical includes 30 or fewer carbon atoms, even more preferably 20 or fewer. Depending on the use to which the compounds are put, it may be desirable that R³ include 10 or fewer, or even 4 or fewer, carbon atoms 4 or fewer. R₃ may be saturated, unsaturated, cyclic, acyclic, alkyl or aromatic. n is preferably 1; m is preferably 2; and b is preferably 1.

Examples of the units of formula A are: 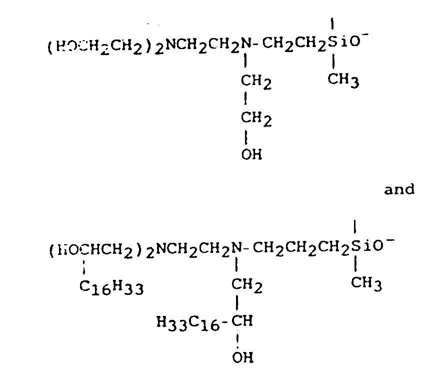
Preferably, the units of Formula A are present in the organosiloxane with units of Formula B:

R⁵_{d}R⁴_{c}SiO_{(4-d-c)/2} B

wherein R⁴ and R⁵ are the same or different monovalent radicals, d and c are integers of 0, 1, 2 or 3 and d plus c is 1, 2 or 3. R⁴ and R⁵ are hydrocarbon radicals, halogenated hydrocarbons, hydrogen, hydroxyl or alkoxyl. Methyl, phenyl and -trifluoropropyl are especially preferred for R⁴ and R⁵. Generally, R⁴ and R⁵ will include from 1 to 20 carbon atoms, preferably from 1 to 10 carbon atoms, more preferably from 1 to 4 carbon atoms. It is particularly preferred that a plus c equal 2.

Preferably, each of the "R" groups described above, e.g., R, R¹, R², etc. includes no greater than 20 carbon atoms, even more preferably no greater than 10 carbon atoms.

The modified aminosilicones of the invention include at least 1 unit of Formula A, and preferably units of Formula B, as well, and may be in the form of random copolymers, block copolymers, linear polymers or branched polymers. The content of Formula A in the polymer ranges by number of repeat units between 0.5% and 100%, preferably between 1% and 10%, more preferably between 5 and 10%. The molecular weight of the modified aminoalkyl silicone preferably ranges from 1000 to 100,000. Molecular weights above 5000 are strongly preferred for a compound which will effectively provide a hydrophobic barrier for a surface. 100,000 is the preferred molecular weight ceiling. A viscosity in the range of 50 10⁻³ Pa.s (cps) to 3000 10⁻³ Pa.s (cps) is preferred.

An Example of the modified polysiloxanes of the invention is as follows:

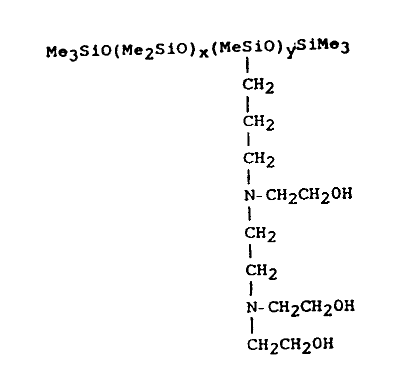

### DETAILED DESCRIPTION OF THE INVENTION:

The compounds of the invention may be prepared by mixing epoxide compounds with aminosilicones in a pressure reactor and heating for about 24 hours, after which the unreacted epoxide compound is vacuum stripped off. The amount of epoxide to be used is calculated based upon the amount of amine functional groups on the aminoalkyl silicone. Preferably, 2 epoxides are reacted for every primary amine and one epoxide for every secondary amine, in order to convert them to tertiary amines. A stoichiometric amount or up to 25% excess of epoxide can be used. The reaction is preferably conducted between 25°C and 150°C, especially between 50°C and 100°C. The pressure is preferably maintained from 50 6.89 kPa (psi) to 300 6.89 kPa (psi); particularly from 50 6.89 kPa (psi) to 150 6.89 kPa (psi). Typical aminosilicone starting compounds would include Dow Corning® Q2-8075.

The compounds of the invention are useful in numerous consumer products which employ silicones, including hard surface cleaners, shampoos, waterproofing agents and car waxes.

Dentifrices which include aminoalkyl silicones of the invention can be used to form a hydrophobic barrier on the surface of teeth which is useful in preventing staining of teeth and in preventing cavities. Yet, the modified compounds tend not to react with aldehydic flavors and other amine-reactive compounds. The antistaining properties of dentifrices incorporating the modified aminosilicones of the invention are of particular significance when the compounds of the invention are used in conjunction with a quaternary ammonium salt such as chlorhexidine. In such event, the dentifrice provides the antistain and anticaries benefit of the aminoalkyl silicone together with the antibacterial and gum disease-fighting benefits of the quaternary ammonium salt, without the usual disadvantage of staining. Anti-calculue effects may also result. The antistaining properties of the amino alkyl silicones may likewise be of use when other staining compounds such as stannous fluoride are included in a dentifrice.

It is our view that the positively charged nitrogen-containing silicones of the invention are attracted to negatively charges surfaces such as enamel so that silicones including alkyl amine groups are more substantive to the surface of teeth and other charged surfaces. Moreover, increasing the number of aminoalkyl groups per molecule enhances the substantivity of the silicone. Also, increasing the charge density improves substantivity, as well. At the same time, modification of the aminoalkyl silicones in accordance with the invention decreases the reactivity of the amino groups toward aldehydes and other reactive groups found in some consumer products including dentifrices.

The modified aminoalkyl silicones of the invention may be used in the form of oils or emulsions.

A preferred class of aminoalkyl silicone are the amodimethicones.

Dentifrices including the compounds of the invention are disclosed in greater detail in Lin et al. application EP-A-0 371 551 filed simultaneously herewith entitled "Dentifrices Including Modified Aminoalkyl Silicones" and incorporated by reference herein.

The modified amines of the invention become protonated and bear positive charges when the pH is below their pKas. Depending on structure, the pKas will range from about 7 to about 9.5.

The aminoalkyl polysiloxanes which are to be modified in accordance with the invention may be end capped. If end capped, one or more of the end capping groups, Rₑ, preferably includes one or more nitrogen atoms. For example, Rₑ may be -(CH₂)₃-NH₂ or -(CH₂)₃-NHCH₂CH₂-NH₂.

As indicated above, a preferred class of aminoalkyl polysiloxanes useful in preparing the compounds of the invention is that of the amodimethicones. Amodimethicones are polydimethyl siloxane polymers containing aminoalkyl groups. The aminoalkyl groups may be present either pendent or at one or more ends of the polydimethylsiloxane chain. The modified amine groups cause the amodimethicone polymer to develop a net positive charge in aqueous systems over a wide range of pH say, from pH 1 to 9. Amodimethicones are commercially available and include Dow Corning® Q2-8075 mentioned above.

The following specific examples further illustrate the invention, but the invention is not limited thereto.

### Alkylation of Amine Functional Silane Monomer

### Example 1

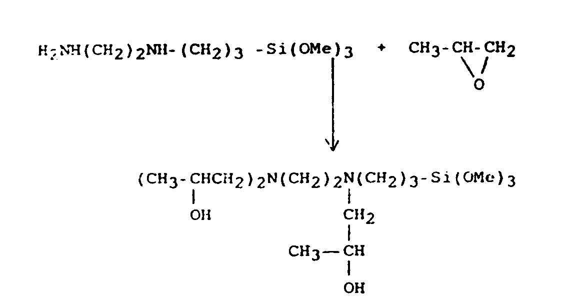

50 g of N-2-aminoethyl-3-amino propyl trimethoxy silane (ex Petrarch), 39.2 g of propylene oxide (ex Aldrich) and 90 g of 2-propanol were reacted together and then stripped of the solvent.

The product was a reactive silicone which precipitated out during the amine titration.

### Alkylation of Amino Functional Polydimethylsiloxane Copolymer

### EXAMPLE 2

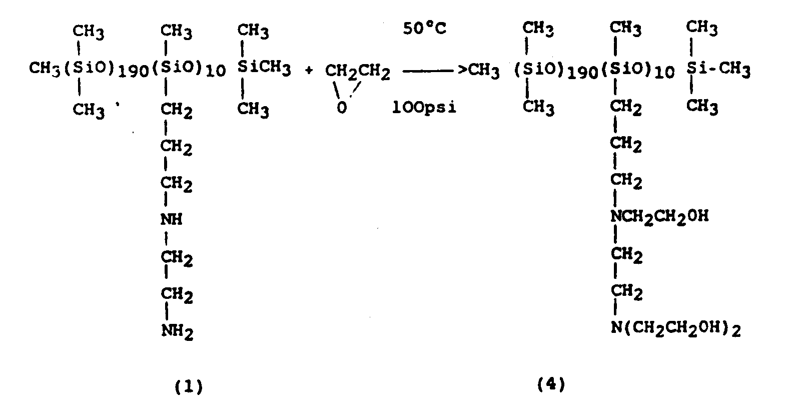

Into a cooled Parr Pressure Reactor was placed 80 g of amodimethicone Oil (1) and 6.5g of ethylene oxide. The contents were then heated at 50°C and 100 psi and stirred for approximately 24 hours. Within 0.5 hours of the start of the reaction, an exotherm was observed at 75°C. After the 24 hour reaction time, the heat was removed and the contents cooled to room temperature. Following this the pressure was released from the reactor and the unreacted epoxide removed by just bubbling through concentrated sodium hydroxide solution followed by evaporation under reduced pressure.

The product was analyzed for amine content (total, secondary plus tertiary, and tertiary) via potentiometric titration, (see Official and Tentative Methods of the American oil Chemists Society; Tf la-64 and Tf 2a-64). As indicated below, 93% of the amine was present in its tertiary form.

### Results of Titration - Example 2:

| | |
|---|---|
| Total amine (1° + 2° + 3°): | 1000 x 10⁻⁶ mol/g |
| Secondary plus tertiary amine (2° + 3°): | 1049 x 10-6 mol/g |
| Tertiary amine (3°): | 929 x 10-6 mol/g |

The results show that 93% of the total mine present was of the tertiary form.

### EXAMPLE 3

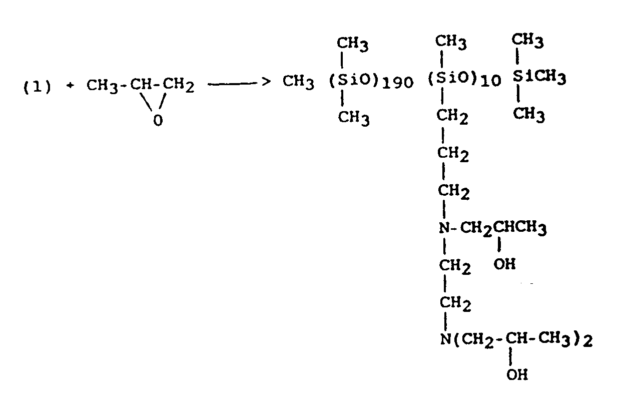

As in Example 2, 250 g of Amodimethicone Oil (1) and 33 g of propylene oxide were reacted initially at 50°C and 100 6.89 kPa (psi) until an exotherm of approximately 70°C was observed. Following the exotherm, the reaction was carried out at 100°C and 100 psi for about 20-24 hours. The product was "stripped" and analyzed as in Example 2.

### Results of Titration - Example 3:

| | |
|---|---|
| Total amine: | 1110 x 10⁻⁶ mol/g |
| Secondary plus tertiary amine | 1077 x 10⁻⁶ mol/g |
| Tertiary amine: | 947 x 10⁻⁶ mol/g |

Approximately 85% of Total amine was converted to tertiary form.

### Example 4

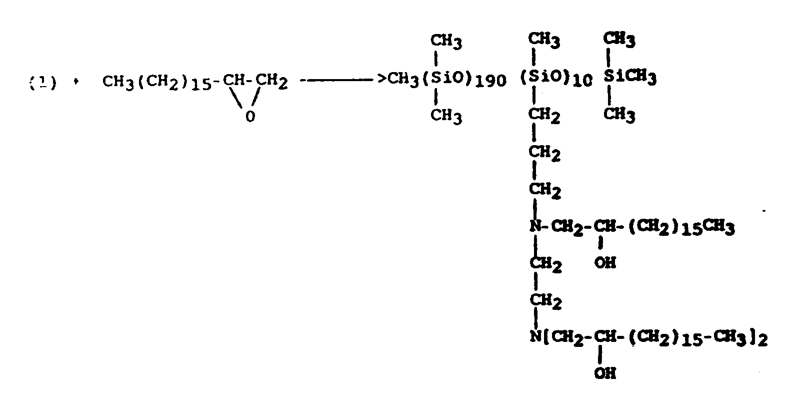

As in Example 4, 65 g of (1), 33 g of 1,2-epoxy-octadecane (ex Aldrich) and 100 g of isopropanol were reacted together. The final product was a solid yellowish "wax" at room temperature which upon heating (45-50°C) becomes a viscous yellowish oil.

The product was analyzed as in Example 2 with one modification being made. It was necessary to use Toluene as a cosolvent together with acetic anhydride and glacial acetic acid since the product was found to be insoluble in a solution of acetic anhydride and glacial acetic acid alone.

### Results of Titration - Example 4

| | |
|---|---|
| Total amine: | 754 x 10⁻⁶ mol/g |
| Secondary plus tertiary amine: | 673 x 10⁻⁶ mol/g |
| Tertiary amine: | 611 x 10⁻⁶ mol/g |

About 81% of the total amine content was converted to the tertiary form.

### Alkylation of Amino Functional Polydimethylsiloxane Copolymer

### Example 5

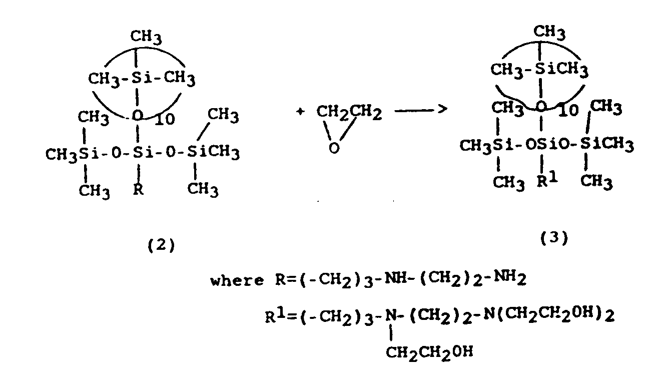

As in Example 2, 100 g of silicone fluid (2), 11.6 g of ethylene oxide and 110 g of 2-propanol were reacted together. The reaction conditions were 50°C and 100 6.89 kPa (psi) for 24 hours. The final stripped product was milky white in appearance. There were two changes made in the analysis of the product. First, chloroform was the solvent chosen for total as well as secondary plus tertiary amine determination since the product was insoluble in a solution of ether and isopropanol. Second, the hydrochloric acid titrant was 0.2N. The same phenomenon, observed in Example 1 where the titrating solution became turbid and formed a precipitate, was observed when titrating this product for total and secondary plus tertiary amine content. The titration showed that all amines were converted to tertiary amines.

### ANTICARIES PROPERTY OF AMINOSILICONES AS DEMONSTRATED BY THE DISSOLUTION TEST OF HYDROXYAPATITE POWDER

### EXAMPLE 6

In these sequential exposure experiments, hydroxyapatite powder (3.5%) was first treated with a 5% silicone emulsion (60-70ml) for ten minutes, filered, washed with distilled water, and then exposed in a pH 5, 150ml acetic acid solution including 5% silicone emulsion. Aliquots were withdrawn at various time intervals and filtered off the hydroxyapatite powder. The amounts of phosphate ion in the aliquots were measured by UV spectrophotometry at 710 nm with molybdate solution in accordance with Official Methods of Analysis, Association of Official Analytical Chemists, edited by Sidney Williams, Arlington, Virginia, p. 632 (1984). The results are shown in Table 1.

The ethoxylated aminoalkyl silicone had the structure of (4) in Example 2.

**TABLE 1**

| ACID DISSOLUTION TESTS OF HYDROXAPATITE POWDER WHICH WAS TREATED WITH AMINOSILICONES | | | | | | | | |
|---|---|---|---|---|---|---|---|---|
| | | 5 Min. | 10 Min. | 20 Min. | 30 Min. | 40 Min. | 50 Min. | 60 Min. |
| Sequential exposure | Ethoxylated Aminoalkyl silicone | 316 | 350 | 370 | 381 | 357 | 386 | 366 |
| | Polydimethylsiloxane | 344 | 358 | 380 | 404 | 380 | 431 | 417 |
| | Untreated HAP | 373 | 432 | 425 | 447 | 444 | 440 | 438 |

This experiment demonstrates that after 60 minutes exposure to the acid solution, the untreated powder lost 438 ppm phosphate ions whereas the one treated with the modified amino alkyl silicone of the invention lost only 366 ppm phosphate ion.

## Claims

1. An organosiloxane comprised of at least one unit of formula A: 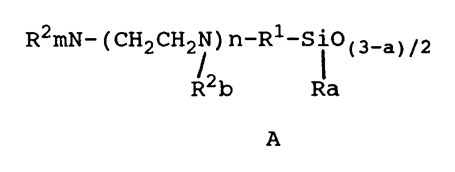 wherein
a is from 0 to 2, n is from 1 to 5, R is a monovalent radical selected from hydrocarbon radicals, halogenated hydrocarbon radicals, hydrogen, hydroxyl and alkoxyl groups, R¹ is a divalent hydrocarbon radical, R² is 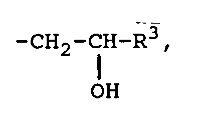 R³ is selected from hydrogen and monovalent hydrocarbon radicals,
m is 1 or 2, and b is 0 or 1, whereby when m = 1 and/or b = 0, the remainder of the valences on the N-atoms are satisfied by H-atoms,
the content of the formula A in the organosiloxane ranging, by number of repeat units, from 0.5 % to 100 %.

2. Organosiloxane of claim 1 wherein said unit is:

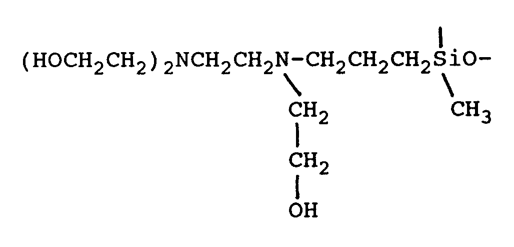

3. The organosiloxane of claim 1 further comprising at least one unit of formula B,
R⁵_{d}R⁴_{c}SiO_{(4-d-c)/2} B
wherein R⁴ and R⁵ are monovalent radicals which can be the same or different selected from hydrocarbon radicals, halogenated hydrocarbon radicals, hydrogen, hydroxyl and alkoxyl groups, d and c are integers of 0, 1, 2 or and d plus c is 1, 2 or 3.

4. The organosiloxane of claim 3 having the formula 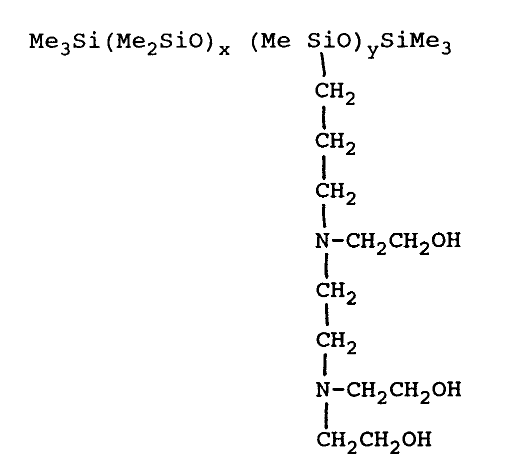 wherein x and y are randomly arranged and x is from 750 to 1250 and y is from 25 to 100.

5. The organosiloxane of claim 3 having the formula 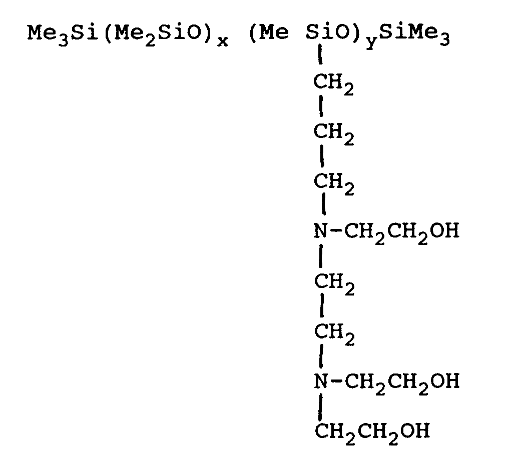 wherein the organosiloxane is a block copolymer and x is from 750 to 1250 and y is from 25 to 100.

## Patentansprüche

1. Ein Organosiloxan, das wenigstens eine Einheit der Formel A umfasst: 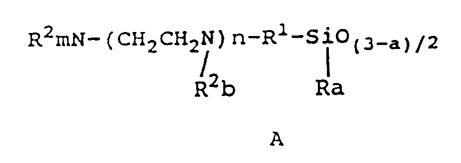 worin
a von 0 bis 2 ist, n von 1 bis 5 ist, R ein monovalenter Rest ist, ausgewählt aus Kohlenwasserstoffresten, halogenierten Kohlenwasserstoffresten, Wasserstoff, Hydroxyl und Alkoxylgruppen, R¹ ein divalenter Kohlenwasserstoffrest ist, R² -CH₂-CHOH-R³ ist, R³ aus Wasserstoff und monovalenten Kohlenwasserstoffresten ausgewählt wird, m 1 oder 2 ist, und b 0 oder 1 ist, wobei, wenn m = 1 und/oder b = 0 ist, der Rest der Valenzen an den Stickstoffatomen durch H-Atome gesättigt ist, wobei der Gehalt an Formel A in dem Organosiloxan für die Anzahl an sich wiederholenden Einheiten im Bereich von 0,5% bis 100% liegt.

2. Organosiloxan von Anspruch 1, worin die Einheit ist:

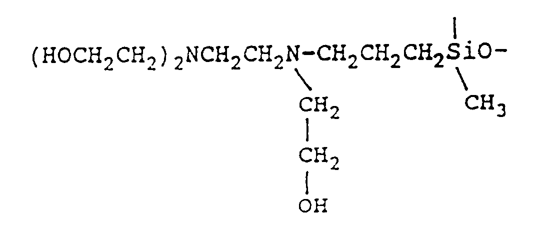

3. Das Organosiloxan von Anspruch 1, das zusätzlich wenigstens eine Einheit der Formel B umfaßt,
R⁵_{d}R⁴_{c}SiO_{(4-d-c)/2} B
worin R⁴ und R⁵ monovalente Rest sind, die gleich oder unterschiedlich sein können, ausgewählt aus Kohlenwasserstoffresten, halogenierten Kohlenwasserstoffresten, Wasserstoff, Hydroxyl und Alkoxylgruppen, d und c ganze Zahlen von 0, 1, 2 oder 3 sind und d plus c 1, 2 oder 3 ist.

4. Das Organosiloxan von Anspruch 3 mit der Formel 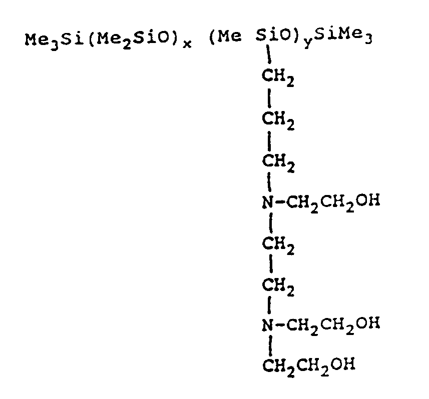 worin x und y statistisch verteilt sind und x von 750 bis 1250 ist und y von 25 bis 100 ist.

5. Das Organosiloxan von Anspruch 3 mit der Formel 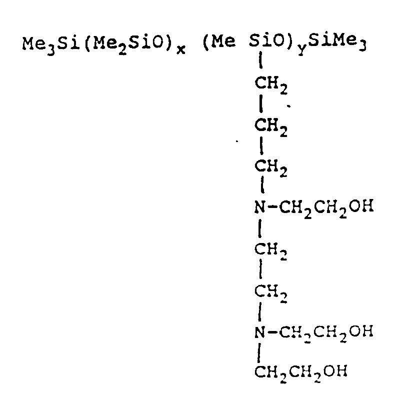 worin das Organosiloxan ein Block-Copolymer ist, und x von 750 bis 1250 ist und y von 25 bis 100 ist.

## Revendications

1. Organosiloxane qui comprend au moins une unité de formule A : 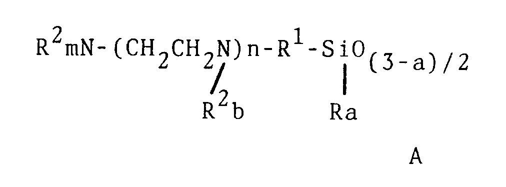 dans laquelle
A est compris entre 0 et 2, n est compris entre 0 et 5, R est un radical monovalent choisi parmi des radicaux hydrocarbures, des radicaux hydrocarbures halogénés, l'hydrogène, des groupes hydroxyles et alcoxyles,
R¹ est un radical hydrocarbure divalent, R² est 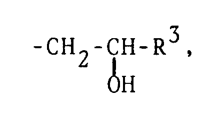 R³ est choisi parmi l'hydrogène et les radicaux hydrocarbures monovalents,
m est 1 ou 2, b est 0 ou 1, tandis que quand m = 1 et/ou b = 0, les valences restantes sur les atomes N sont occupées par des atomes H,
la teneur de la formule A dans l'organosiloxane, en nombre d'unités répétées est comprise entre 0,5% et 100%.

2. Organosiloxane de la revendication 1 dans lequel ladite unité est

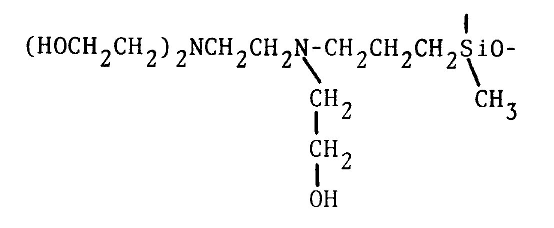

3. Organosiloxane de la revendication 1 qui comprend en plus au moins une unité de formule B,
R⁵_{d}R⁴_{c}SiO_{(4-d-c)/2} B
dans laquelle R⁴ et R⁵ sont des radicaux monovalents qui peuvent être identiques ou différents, choisis parmi les radicaux hydrocarbures, les radicaux hydrocarbures halogénés, l'hydrogène, les groupes hydroxyles et alcoxyles, d et c sont des entiers valant 0, 1, 2 ou 3 et d + c représentent 1, 2 ou 3.

4. Organosiloxane de la revendication 3 qui a la formule 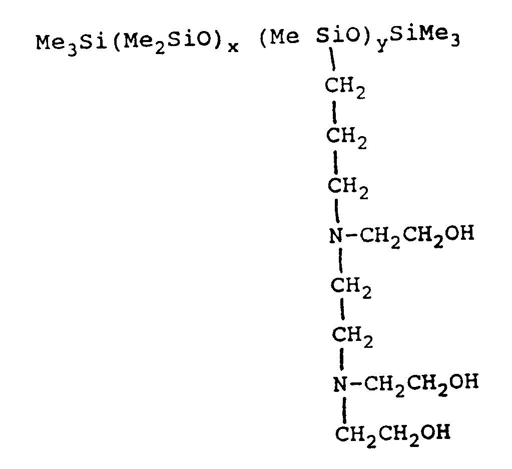 dans laquelle x et y ont des valeurs aléatoires et x est compris entre 750 et 1250 et Y est compris entre 25 et 100.

5. Organosiloxane de la revendication 3 qui a la formule 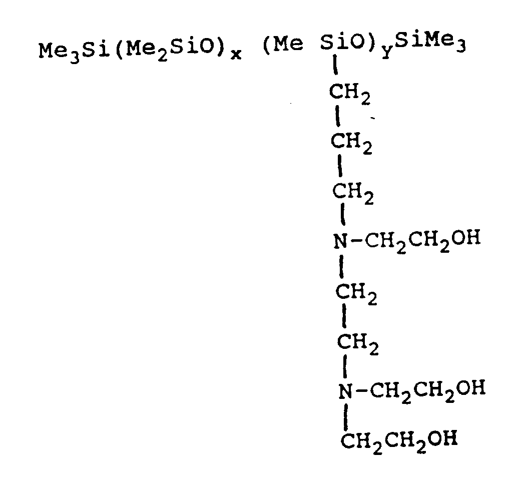 dans laquelle l'organosiloxane est un bloc copolymère et x est compris entre 750 et 1250 et y est compris entre 25 et 100.
